# EUROPEAN PATENT SPECIFICATION

(11) **EP 1 844 788 B1**
(45) Date of publication and mention of the grant of the patent: **14.07.2010**
(21) Application number: 06007834.2
(22) Date of filing: 13.04.2006
(51) Int. Cl.: A61K 39/00, A61K 39/385, A61K 39/39, A61P 35/00

(54) **HER-2/neu multi-peptide vaccine**
HER-2/neu Multipeptidimpfstoff
HER-2/neu vaccin à base de multiples peptides

(43) Date of publication of application: 17.10.2007
(73) Proprietor: Bio Life Science Forschungs- und Entwicklungsges.m.b.H., 1010 Wien (AT)
(72) Inventor: Zielinski, Christoph, 1180 Vienna (AT); Schreiner, Otto, 2380 Perchtoldsdorf (AT); Pehamberger, Hubert, 1230 Vienna (AT); Breiteneder, Heimo, 1070 Vienna (AT); Wiedermann, Ursula, 1130 Vienna (AT)
(74) Representative: Kador & Partner

(56) References cited:
- EP-A- 1 236 740
- WO-A2-01/08636
- JP-A- 5 117 165
- KAUMAYA PRAVIN T P: "HER-2/neu cancer vaccines: Present status and future prospects" INTERNATIONAL JOURNAL OF PEPTIDE RESEARCH AND THERAPEUTICS, vol. 12, no. 1, March 2006 (2006-03), pages 65-77, XP002406488 ISSN: 1573-3149
- DAKAPPAGARI N K ET AL: "A chimeric multi-human epidermal growth factor receptor-2 B cell epitope peptide vaccine mediates superior antitumor responses" JOURNAL OF IMMUNOLOGY, THE WILLIAMS AND WILKINS CO. BALTIMORE, US, vol. 170, no. 8, 15 April 2003 (2003-04-15), pages 4242-4253, XP002290185 ISSN: 0022-1767
- DISIS MARY L ET AL: "Generation of T-cell immunity to the HER-2/neu protein after active immunization with HER-2/neu peptide-based vaccines." JOURNAL OF CLINICAL ONCOLOGY : OFFICIAL JOURNAL OF THE AMERICAN SOCIETY OF CLINICAL ONCOLOGY. 1 JUN 2002, vol. 20, no. 11, 1 June 2002 (2002-06-01), pages 2624-2632, XP002406489 ISSN: 0732-183X
- SALAZAR L G ET AL: "Immunization of cancer patients with HER-2/neu-derived peptides demonstrating high-affinity binding to multiple class II alleles" CLINICAL CANCER RESEARCH, THE AMERICAN ASSOCIATION FOR CANCER RESEARCH, US, vol. 9, no. 15, 15 November 2003 (2003-11-15), pages 5559-5565, XP002293866 ISSN: 1078-0432
- WAGNER S ET AL: "Immunization with a multiepitope peptide vaccine and co-administration of IL-12 prevents tumor growth in Her-2 transgenic mice" BREAST CANCER RESEARCH AND TREATMENT, vol. 88, no. Suppl. 1, 2004, pages S243-S244, XP002406490 & 27TH ANNUAL CHARLES A COLTMAN SAN ANTONIO BREAST CANCER SYMPOSIUM; SAN ANTONIO, TX, USA; DECEMBER 08 -11, 2004 ISSN: 0167-6806

## Description

The present invention relates to a multi-peptide vaccine against cancerous diseases associated with the HER-2/neu oncogene.

The tumor antigen HER-2/neu, gene product of erbB2/neu protooncogene, is a 185 kDa protein that belongs to the epidermal growth factor receptor family. It consists of a cysteine rich extracellular domain (ECD) with several glycosylation sites, a hydrophobic transmembrane domain, and an intracellular conserve tyrosine kinase domain. HER-2/neu is weakly detectable in epithelial cells of normal tissues but is overexpressed in 20 to 30 % of primary breast, ovarian, colon, lung and prostate cancer, and has been linked with poor prognosis and high risk of cancer relapse. The overexpression seems to be stable and homogenous in primary tumors as well as their metastases. Hence, HER-2/neu is an attractive target of cancer immunotherapy.

Passive immunotherapy with monoclonal antibodies is a routinely performed treatment against cancer diseases. Induction of humoral anti-tumor antibody responses by active peptide immunization has therefore become a favourable treatment concept. At present, therapy with Trastuzumab, also known as Herceptin^{®}, a Her-2/neu specific monoclonal antibody (mAb), is standard for the treatment of advanced breast cancers overexpressing the Her-2/neu oncoprotein. The therapeutic efficacy of this mAb suggests that targeting tumors with antibodies has a significant preventive activity. Nevertheless, the utility of passive immunotherapy is limited by inadequate tissue distribution and the necessity of multiple infusions with associated high costs.

Nowadays there are several vaccine strategies targeting HER-2/neu. Such a vaccine strategy requires the development of a method to overcome immune tolerance to the self-protein HER-2/neu.

EP1236740 refers to a vaccine comprising two peptides with a length of 13 and 16 amino acids and a sequence, which occur in the extracellular domain of HER-2/neu protein. The used peptides permit active immunization against cancerous diseases associated with HER-2/neu oncogene, but induce less inhibition of tumor growth than Trastuzumab.

Therefore, object of the present invention is to provide a vaccine against cancerous disease involving an overexpression of HER-2/neu protein, which induces strong immunity to establish a immune memory and thus to avoid the disadvantages of conventional cancer treatments and to offer a convincing alternative to other known vaccination methods. A further object of the present invention is to provide a vaccine, which can be administrated to the patient via oral and nasal (mucosal) routes without loosing its strong immunogenic property.

The present invention is based on the finding that the above object can be achieved if the vaccine is a multi-peptide - multiepitop - vaccine against cancerous diseases associated with HER-2/neu oncogene, i.e. the vaccine comprises a specific combination of peptides presenting different amino acids sequences as occur in the extracellular domain of HER-2/neu protein.

Furthermore, it has been found that in order to improve the immunity response the vaccine should optionally comprises an immune-stimulation adjuvant or delivery system, in case the vaccine should administrated via oral or nasal route without loosing its strong immunogenic property, the vaccine optionally comprises a mucosal adjuvant or a mucosal antigen delivery system which is used as mucosal carrier.

Therefore, the present invention provides a vaccine against cancerous diseases associated with the HER-2/neu oncogene, wherein said vaccine comprises a mixture of at least three different peptides having a length of 9 to 30 amino acids and each sequences occurs in the extracellular domain of HER-2/neu protein, wherein at least one of the three peptides has the sequence 378 to 394 of the extracellular domain of HER-2/neu protein, at least another one has the sequence 545 to 560 of the extracellular domain of HER-2/neu protein and at least a further peptide has the sequence 610 to 623 of the extracellular domain of HER-2/neu protein, and wherein each of the peptides is conjugated to a carrier.

The invented vaccine induces a strong immunity and establishes an immuno-memory against cancerous diseases associated with the HER-2/neu oncogene. Thus, the vaccine provides prophylaxis against these cancerous disease. In addition, the inventive vaccine can be used to treat an already existing cancerous disease or to accompany conventional cancer treatments. Application of the inventive vaccine can completely or partly avoid the above-described considerable disadvantages of conventional/passive cancer immunotherapeutics.

In particular, the peptide having the sequence from 378 to 394 of the extracellular domain of HER-2/neu protein is ID SEQ 1: PESFDGDPASNTAPLQP.

The peptide having the sequence from 545 to 560 of the extracellular domain of HER-2/neu protein is ID SEQ 2: RVLQGLPREYVNARHC.

The peptide having the sequence from 610 to 623 of the extracellular domain of HER-2/neu protein is ID SEQ 3: YMPIWKFPDEEGAC.

The inventive peptides can also be linked to other peptides or polypetides or to further chemical groups such as glycosyl groups, lipids, phosphates, acteyl groups or the like, as e.g. polyglycol, polyethylengycol, poly-lactic acid (PLA), poly-lactic-co-glycolic acid (PLGA), lysine dendrimers. These substances do not adversely influence the biologically activity of the peptides.

It is preferred that peptide ID SEQ 1 is additionally linked to any linker known in the art, but preferably to a glycine linker and/or a C-terminal cysteine residue, more preferably to the linker of GGGGGC.

Therefore, the preferred vaccine of the present invention comprises a mixture of peptide ID SEQ 1 linked to a glycine linker and/or a C-terminal cysteine residue, in particular to the linker GGGGGC, and peptide ID SEQ 2 as well as peptide ID SEQ 3.

Moreover, it is preferred that each of the inventive peptides are conjugated to a carrier, particularly for systemic immunization.

The used peptides may be conjugated by every method known in the art, for example by genetic engineering or by chemical means, which includes linking of carrier and functional group by a chemical reaction. By genetic engineering, conjugation of the carrier, which may a protein molecule, to the peptide can be effected by inserting a DNA or RNA sequence coding for the total sequence of the conjugate into the an expression system which then expresses the total conjugate.

Additionally, the peptides may be linked to the carrier via a further linker. Thereby, the linker acts as a spacer that confers flexibility or, if desired, rigidity of the conjugated peptide. The chemical nature of the spacer may vary, depending on the reactivity of the functional groups of the carrier and the peptide, respectively, and depending on the necessity in respect of flexibility or rigidity. As an example, spacing sequences such as C-terminal cysteine residues, or glycine like (G)ₓ may be mentioned.

Each of the inventive peptides can be conjugated to the carrier in a single or multiple way in different combinations as mono-, di-, tri or oligomers. Such conjugations are described for example in the publication by Th. H. Turpen, S.J. Reinl, Y. Charoenvit, S.L. Hoffmann, V. Fallarme in Bio/Technology, 1995, Vol. 13, pages 53 to 57, by examples of the conjugation of epitopes to macromolecular carriers, or by Wagner et al, 2005 J. Immunol. 174:976-982.

It is preferred that the carrier itself has an immune effect, which means the carrier itself is immunogenic.

The carrier is selected from the group consisting of immunogenic peptides, immune stimulation protein sequences like GPC islands, limpet hemocyanin (KLH), tetanus toxoid (TT), cholera toxin subunit B (CTB), bacteria or bacterial ghosts, liposome, chitosome, virosomes, microspheres, dendritic cells, or their like.

In one preferred embodiment each inventive peptide is preferably conjugated to KLH, TT or CTB as carrier by a chemical reaction.

In another preferred embodiment each inventive peptide is preferably conjugated to a virosome or probiotic lactic acid bacteria (LAB) or bacterial ghosts as carrier.

Virosomes are based on liposomes and contain viral proteins embedded in their membranes. These proteins enable the virosome membranes to fuse with cells of the immune system and thus, deliver their contents - the vaccine-specific antigens, in this case the inventive peptides, directly to their targets. Once the virosomes delivered the antigens, the virosomes are completely degraded within the cells. The origin of the virosomes may be a influenza virus.

Furthermore, it is preferred that the vaccine comprises an adjuvant. Adjuvants are biological substances, which enhance humoral and/or cellular immune responses when given with vaccine antigen, in this case with the inventive peptides. In the present invention it is preferred that the adjuvant is used for a systemic immunization.

The adjuvants are selected from the group consisting of e.g. interleukines, bacterial toxins, bacterial cell walls and particles thereof, lipidparticles, aluminiumhydroxide, squalen derivate, monophospharyl lipid A, or their like.

Moreover, it is preferred that the vaccine can be administrated oral or nasal routs. However, most of protein and/or peptide antigens including purified vaccine antigens are often poorly immunogenic when administered via oral and nasal routes. Thus, co-administeration of mucosal adjuvants are essential to induce effective immune responses.

The advantage of a mucosal vaccine lies in a better compliance of the patient to vaccination and a potentially higher efficacy due to the induction of both systemic and mucosal immune responses, this is also e.g. important for tumors situated at the mucosae.

The mucosal adjuvants might be selected from the group consisting of zonula occuldens toxin, heat labile toxin (LT) produced by enterotoxigenic E.coli, vibrio cholerae cholera toxin (CTB) or non-bacterial origin e.g. liposomes, or their like.

A preferred mucosal adjuvant is cholera toxin B subunit, lactic acid bacteria, bacterial ghost or mutant thereof.

In the present invention it is further preferred that each of the inventive peptide is conjugated to the mucosal adjuvant, i.e. each of the inventive peptide is coupled to the mucosal adujuvant for example by a chemical reaction and not only mixed with the mucosal adjuvant. Hence the mucosal adjuvant acts as carrier for the peptides.

In a further preferred embodiment of the present invention probiotic lactic acid bacteria (LAB) are used as mucosal carriers, in particular as mucosal antigen delivery systems. It is known that some lactic acid bacteria (LAB) possess Th1 promoting properties and have therefore been used for vaccination against the Th-2 biased allergic diseases (Repa et al Vaccine 2002; Daniel et al 2006, Allergy).

In the present invention it is shown that IL-12, promoting Th1 responses, enhance the anti-tumor activity of the multi-peptide vaccine. It is therefore preferred to apply certain lactic acid bacteria, which induce IL-12 induction and Th-1 responses, together with the polypeptide vaccine of the present invention. Of particular interest is to use these bacteria as expression system for production of the peptides and also use them as mucosal delivery system. Thereby an oral/mucosal tumor vaccine can be created.

In particular, in the present invention strains of *Lactobacillus plantarum* or *Lactococcus lactis* are preferably used as mucosal carriers.

The production of such mucosal carries and the production of vaccines including such carriers are described e.g. in Repa et al., Vaccine, 2003, 22, pages 87 to 95 or Daniel et al, Allergy, 2006.

Furthermore, it is preferred that an further immunogenic adjuvant is added to the inventive vaccine, more preferably interleukine 12 (IL-12) or an IL-12 agonist or a substance that promotes IL-12 production.

Moreover, the vaccine of the present invention may further comprises additives, which are general used in such application like stabilizer, antidegradant etc.

The inventive vaccine can be produced in diverse ways by genetic engineering or chemical means, e.g. solid-phase synthesis method. Such methods are described for example in US 5, 869,445.

An example of a genetically engineered production method is to manipulate microorganismen like E. coli or the above mentioned lactic acid bacteria. These are manipulated so that they express the peptides as such or the total conjugates consisting of peptide and carrier coupled thereto.

The inventive vaccine can also be applied in different ways. The vaccine can be administrated for examples intramuscularly, subcutanesously, orally, intranasally or generally mucosally if the vaccine is in capsule or pill form or dispersed in food like yoghurt, if lactic acid bacteria are used as vaccine carriers, or a specific delivery system is used. If the vaccine contains functional nucleic acid variants of the peptides, it can also be administrated by an ex-vivo procedure, which includes removing cells from an organism, inserting the inventive vaccine into these cells and placing the treated cells back in the organism.

The inventive vaccine with or without co-administration of IL-12 can be administrate to the patient according to any treatment schedule.

However, a repeated treatment with IL-12 in a three week interval alone did not show any beneficial or deleterious effect as it has been reported for a treatment in a one week interval (Boggio et al., J. Exp. Med 1998; 188: 589 - 96). Therefore, it is preferred that three inventive peptides are given to the patient 4 times in 14 to 21 days interval and further that a day after the three peptides were given a five-day course of IL-12 co-application or an IL-12 agonist or a formulation that induces IL-12 production follows, wherein at the first two days IL-12 is given in a lower concentration than in the last three days. It is preferred that the first concentration of IL-12 is half as high the second one.

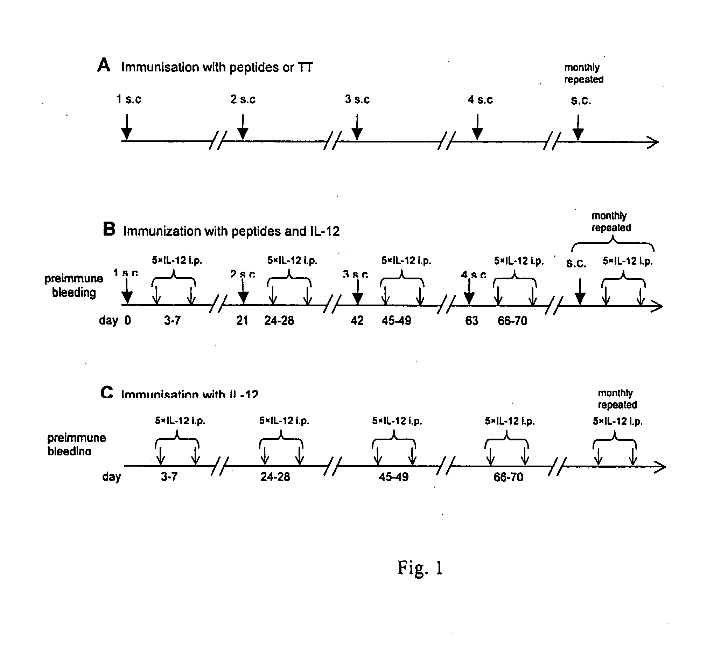

The inventive vaccine can be used for prophylactic or acute treatment of mammals that can develop kinds of cancer associated with the HER-2/neu oncogene or in combination with other chemotherapeutics or for prevention of metastasis following surgical intervention.
Fig. 1: Experimental design: MMTV-c-*neu* mice (i.e. Her-2/neu transgenic mice, which spontaneously develop breast cancer) were repeatedly immunized with A) the HER-2/neu peptide conjugates (15 µg each) or TT-alone, B) co-administration with IL-12 or C) with IL-12 alone, in monthly intervals until sacrifice. In separate set-up BALB/c mice were immunized as described for MMTV-c-*neu* mice and were sacrificed 10 days after the forth immunization.
Fig. 2 : Immunoprotective effects of HER-2/neu specific vaccination on tumor formation and tumor progression in MMTV-c-neu transgenic mice.
   Upper panel: The time to tumor development was analysed using Kaplan-Meier survival analysis. Mice (n = 8) were vaccinated with TT-conjugated HER-2/neu peptides (0), TT-conjugated HER-2/neu peptides and IL-12 co-application (◆), TT alone (■), IL-12 alone (n=5, o) according to the experimental design given in the Fig. 1. The control group (•) remained untreated. Lower panel: Tumor progression expressed in weeks until the cumulative tumor volume reached 1000 mm³ /mouse. * p < 0.05.

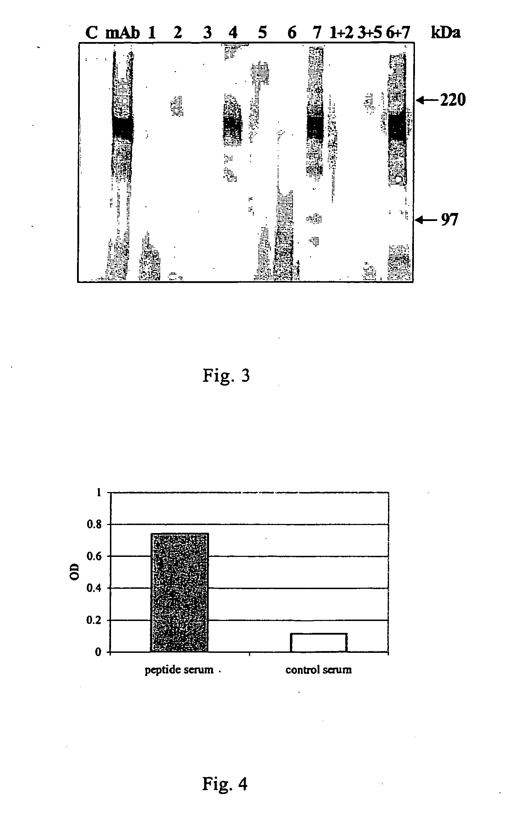

Fig. 3: SK-BR-3 cell lysates were incubated with sera of mice immunized with adjuvant and TT as a control group (lane C), with a murine monoclonal anti human Her-2/neu Ab as a positive control (lane mAb), with sera of mice immunized with conjugated peptides P1 - P3, P5 and ID SEQ 1 to 3 (lanes 1-7, lane 4 refers to ID SEQ 1, lane 6 refers to ID SEQ 2 and lane 7 to ID SEQ 3) or a combination of P1+P2 (lane 1+2), P3+P5 (lane 3+5), and ID SEQ2 + ID SEQ 3 (lane 6+7). Precipitated Her-2/neu was detected by rabbit anti human Her-2/neu Ab and AP-conjugated swine anti rabbit Ab.
Fig. 4: Her-2/neu specific antibodies measured by ELISA. Peptide serum = sera of mice immunized with peptide/tetanus-conjugate; control serum = sera of mice immunized with tetanus-toxoid.

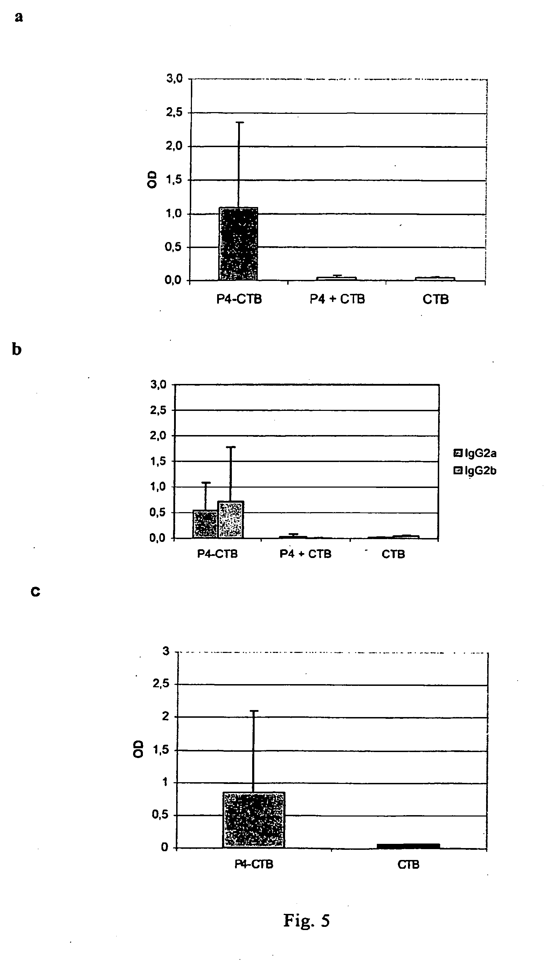

Fig 5: Immunisation of the mice with the peptide conjugate of ID SEQ 1 and CTB (P4 refers to ID SEQ 1) or a mixture of ID SEQ 1 and CTB (Fig. 5a); the serum dilution was for IgG1 1:10 000, for IgG2a und IgG2b 1:2000 (Fig. 5 b); IgA in bronchoalvelar lavages (1:1) (Fig. 5c)

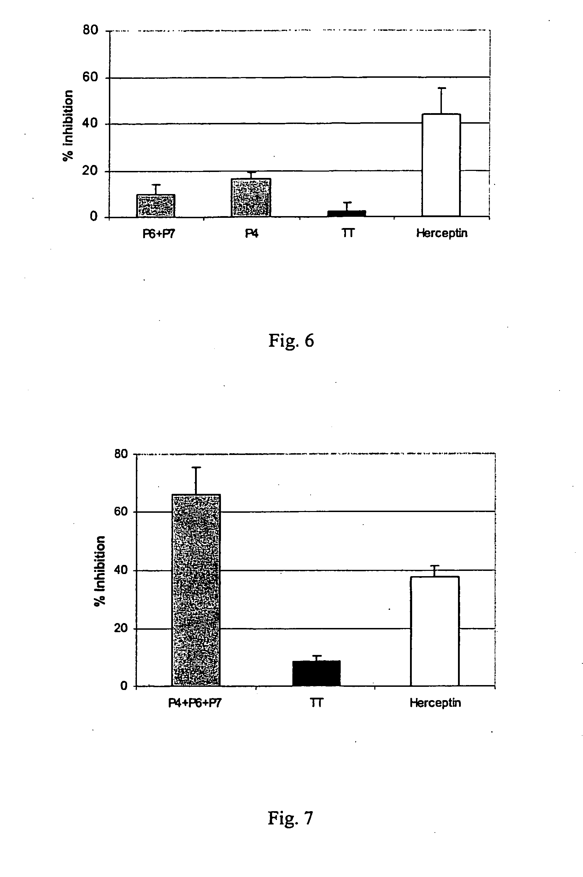

Fig. 6 : Inhibition of SK-BR-3 cells with IgG anti ID SEQ 2 and 3 (called P6 and P7) and anti ID SEQ 1 (called P4) and anti TT with a concentration of 150µg/ml , Trastuzumab (Herceptin) with a concentration of 50 µg/ml.
Fig. 7: Inhibition of SK-BR-3 cells with IgG anti ID SEQ 1 and 2 and 3 (called P4, P6 and P7) and anti TT with a concentration of 75µg/ml Trastuzumab (Herceptin) with a concentration of 50 µg/ml.

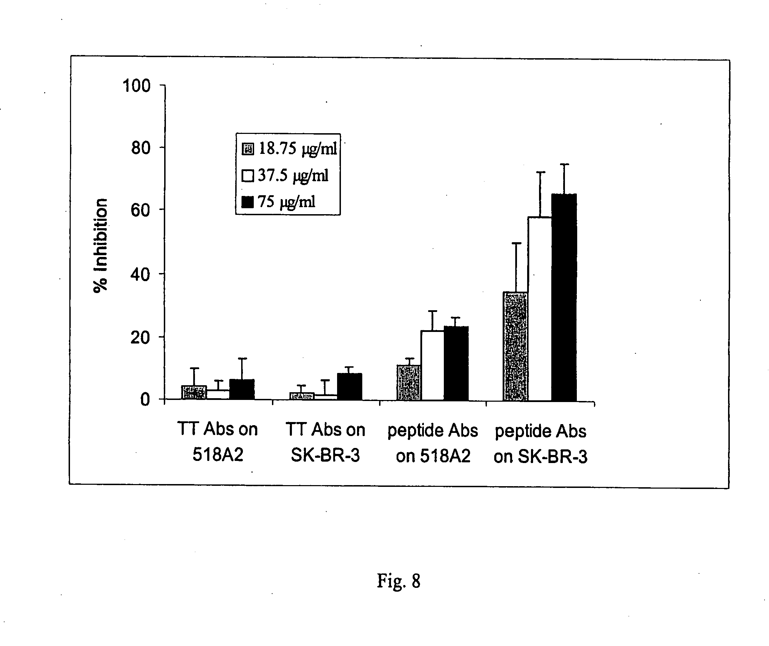

Fig.8: [3H]-thymidine proliferation assay demonstrating the inhibiting effect in a dose dependent manner of the rabbit IgG on SK-BR-3 cell growth. Data are expressed in percentage of inhibition; cpm values of untreated wells were put to 100%.

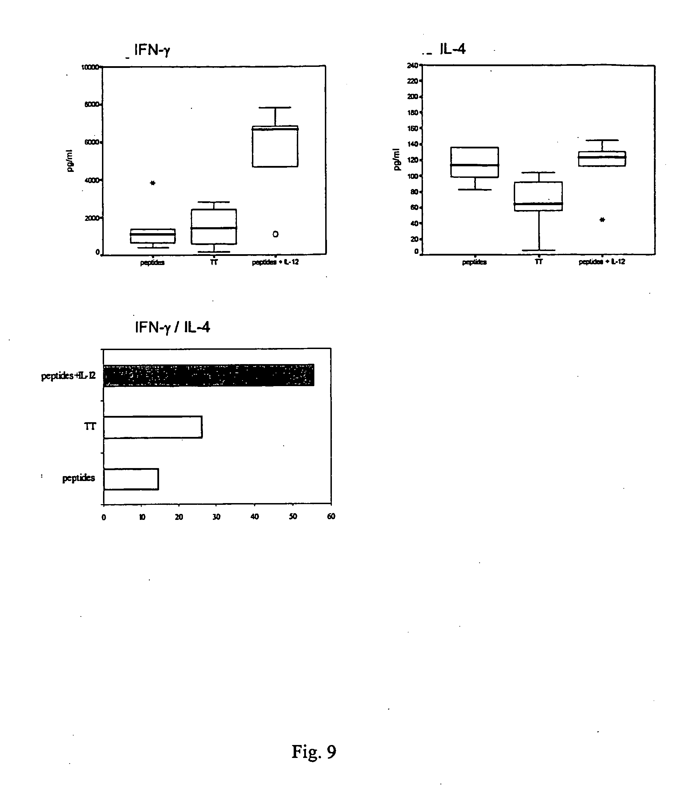

Fig.9: Cytokine production by spleen cells after in vitro stimulation with TT. BALB/c mice (n=5 per group) were immunized with peptides conjugated to TT with or without systemic co-administration of IL-12 or with TT alone. Splenocytes were cultured with TT and cytokine concentrations in supernatants were evaluated by ELISA. A: IFN-γ levels; B: IL-4 levels; C: IFN-γ/IL-4 ratio. Asterisk indicates p<0.05 (Turkey - Kramer test).

### Method and Examples

### Mice

The used female FVB/N mice transgenic for the activated rat *c-neu* oncogene (MMTV-*c-neu,* 5-9 wk old) and BALB/c mice (8 week old) were purchased from Charles River (Sulzfeld, Germany). Overexpression of the c-neu oncogene was driven by a mouse mammary tumor virus (MMTV) promoter and these mice transgenic for the activated rat *c-neu* oncogene develop spontaneously mammary tumors by ∼30 weeks of age. Mammary glands were inspected weekly for tumor appearance and progression.

### Tumors

Tumors were measured with a caliper and the volume was calculated by: x² × y/2, whereby x and y represent the short and long dimensions of the tumor. Total tumor volume per mouse was calculated by adding all tumor volumes. Progressively growing masses of >3mmx3mm were regarded as tumors. Mice were sacrificed for ethical reasons at the time when a total tumor volume of approximately 2000 mm3 was exceeded. Tumors were excised and stored at -80°C.

All experiments were authorized by the Animal Experimentation Committee of the Medical University of Vienna and the Austrian Ministry of Education, Science and Culture.

### Antigens

The protein sequence of the extracellular domain (ECD) of human HER-2/neu was scanned by computer-aided prediction to search for immunogenic epitopes based on hydrophilicity, accessibility, flexibility, charge distribution or secondary structure propensities. Seven peptides, 14 to 21 amino acids in length, were then synthesized with an additional C-terminal cysteine residue N-alpha-fluorenylmethyloxycarbonyl (Fmoc) chemistry by PiChem (Austria).

**Table 1: Tested peptides**

| *Peptide* | *Sequence* | *Amino acids* |
|---|---|---|
| P1 | 115 - 132 | AVLDNGDPLNNTTPVTGA |
| P2 | 149 - 162 | LKGGVLIQRNPQLC |
| P3 | 274 - 295 | YNTDTFESMPNPEGRYTFGAS |
| ID SEQ 1 | 378 - 394 | PESFDGDPASNTAPLQP |
| P5 | 489 - 504 | PHQALLHTANRPEDE |
| ID SEQ 2 | 545 - 560 | RVLQGLPREYVNARHC |
| ID SEQ 3 | 610 - 623 | YMPIWKFPDEEGAC |

Furthermore, for the experiments the peptide ID SEQ 1 (PESFDGDPASNTAPLQP) was synthesized with an additional glycine linker and a C-terminal cysteine residue, in particular with the GGGGGC linker.

### Conjugation of peptides

Peptides were coupled to the carrier proteins tetanus toxoid (TT) or keyhole limpet hemocyanin (KLH) or the B subunit of cholera toxin (such as included in the cholera vaccine Dukoral^{®}) using the heterobifunctional cross-linker reagent m-Maleimidobenzoyl-N-hydroxysuccinimide (MBS) [Pierce, Rockford, IL]. The carrier in the mucosal vaccine are coupled to the peptides according to Wagner S et al 2005; J. Immunol 174:976-982. The amino groups of the carrier proteins were first activated by addition of a 25-fold molar excess of MBS for 30 min at room temperature. Excess MBS was removed by a desalting column (PD-10 column-Amersham Bioscience, Little Chalfont, UK). In a second step peptides were added in a molar ratio of 40:1 peptide-to-carrier protein. Crosslinking occurred to the cysteine residues on the peptides within 3 hours at room temperature. Unbound peptides were removed by dialysis against PBS.

### Vaccination

1. Balb/c Mice (n=5/group) were immunized with three inventive peptides. These peptides were injected individually (25 µg/mouse) or in combination of three peptides (30 µg/mouse). Prior to injection the peptide-conjugates were mixed with Gerbu Adjuvant (Gerbu Technik, Germany) according to the manufacturer's instruction and administered subcutaneously in volume of 100 µl. The control group received the used Carriers and adjuvants alone. The immunizations were performed 4 times in 21 day intervals. Seven days after the last immunization the animals were sacrificed. Blood samples from mice were taken by tail bleeding prior to immunization and seven days after the last immunization.
2. BALB/c Mice (n=5/group) and MMTV-c-neu trangenic mice (n=8/group) were immunized with the combination of the three inventive peptides coupled to tetanus toxoid (TT-conjugates) using 15 µg of each peptide conjugate. Control groups received TT or IL-12 alone or remained unimmunized. Prior to injection, antigens were mixed with Gerbu Adjuvant (Gerbu Technik, Gaiberg, Germany) according to the manufacturer's instruction and administered subcutaneously in a volume of 100 µl. Recombinant mouse IL-12 (Strathmann Biotec, Hamburg, Germany), reconstituted in PBS containing 0.01% mouse serum albumin (Sigma-Aldrich, St. Louis, MO), was administered intraperitoneally in a volume of 100 µl. A total of 50 ng of IL-12/injection was given during the first two five-day-courses, followed by 100 ng of IL-12/injections in the subsequent boosts. Immunizations and IL-12 treatments were performed according to the scheme given in Fig. 1. Blood samples were taken by tail bleeding prior to immunization and at the sacrifice. BALB/c mice were sacrificed ten days after the fourth immunization. MMTV-*c-neu* mice were repeatedly boosted in one-month intervals until sacrifice.
3. Rabbits were immunized with the multi-peptide vaccine or tetanus toxoid and thereafter blood were taken by puncture of an ear-vene prior to and after immunization. Immunizations with the mixture of the peptides, were performed in rabbits at the laboratories of Charles River (Kissleg, Germay). According to the schedule performed in mice the immunizations were done 4 times in 14-21 day intervals. The blood samples were used for in vitro assays.

Spleens, hearts, livers, kidneys and lungs from mice were removed for histopathological analyses.

### Vaccination and tumor development

The ability of the inventive vaccine and the effect of co-application of IL-12 to delay spontaneous tumor development and tumor progression was investigated in transgenic MMTV-*c-neu* FVB/N mice expressing an activated form of rat *c-neu* that results in a rapid tumor development. The immunization schedule was started with 6-10 weeks old mice according to the experimental design given in Fig. 1 with coupled Her-2/neu peptides combined with or without systemic IL-12 treatment. Mice were boosted monthly until sacrifice. Mammary glands were monitored weekly for number and size of tumors.

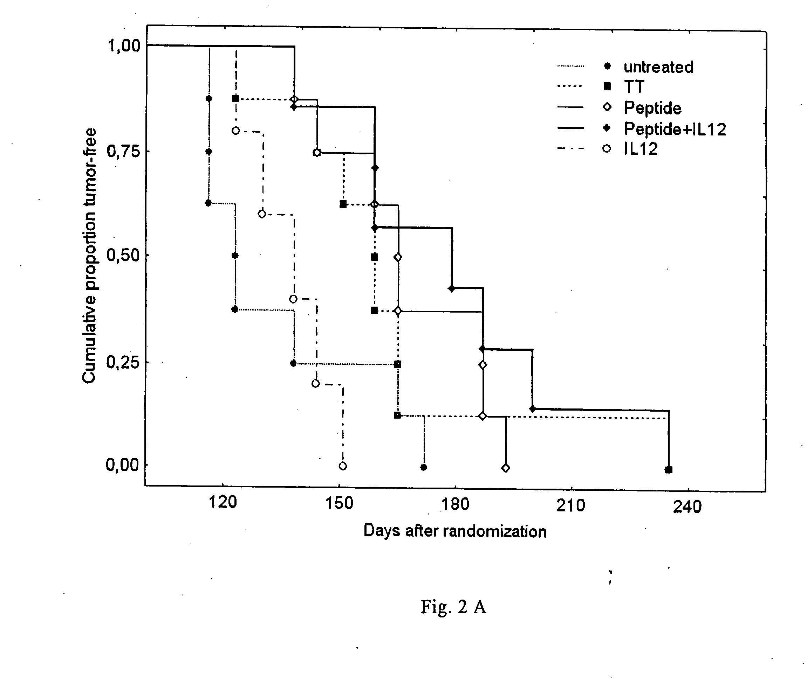

First tumors were observed in untreated and control mice receiving IL-12 alone at the age of 22-26 weeks. Within eight weeks (30-34 weeks of age) all mice in these groups developed tumors whereas a significant delay (p<0.05) in tumor onset was observed in the vaccinated groups (Fig. 2A): in 37.5% (3/8) of peptide-immunized mice and in 57.1% (4/7) of the peptide + IL-12 immunized mice the tumor free interval was prolonged by 56 days. In contrast, in the group receiving the carrier protein TT alone only one mouse remained tumor free during this time period.

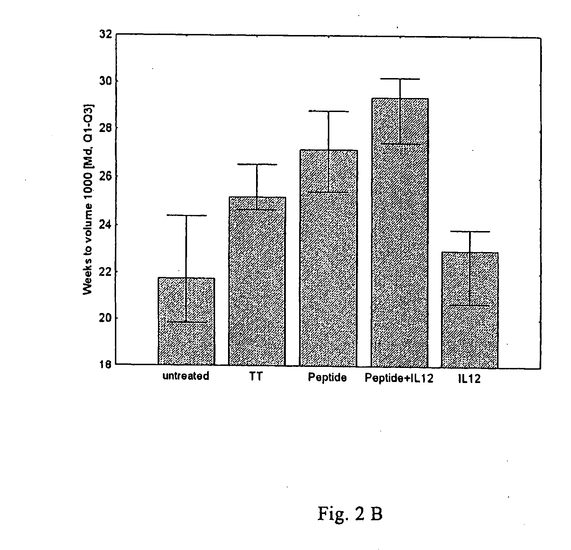

Monitoring of tumor volumes revealed rapid tumor progression in non-immunized controls. In contrast, peptide-vaccinated groups were characterized by slower progression of established tumors in early stage. As indicated in Fig. 2B, non-immunized controls reached a total tumor volume of 1000 mm³ within 21.5 weeks following the first immunization. This period was exceeded for peptide-immunized mice up to 6.5 weeks (27 weeks after first immunization; p=0.08). In mice treated with the peptide-vaccine co-administered with IL-12 the time period until tumor volume of 1000 mm³ was significantly (p<0.05) prolonged up to 8.5 weeks (29 weeks after first immunization) compared to control mice. No significant reduction in tumor progression was observed in the group of mice receiving IL-12 or TT alone, reaching 1000 mm³ tumor volumes with 23 or 25 weeks after first immunization, respectively.

### Cell lines

The human breast cancer cell lines SK-BR-3 (HTB 30) and HTB 132 were purchased form ATCC (Manassas, Virginia, USA). The human melanoma cell lines 518.A2 were kindly provided from B. Jansen (Department of Dermatology, University of Vienna, Austria). The murine mammary tumor cell line Tg1-1 was kindly provided by T.J. Kipps (Division of Hematology/Oncology, University of California, San Diego School of Medicine, CA). Cells were cultured in medium containing 10 % fetal bovine serum (PAA Laboratories, Linz, Austria), 50 units/ml penicillin and 50 µg/ml streptomycin (GIBCO, Life Technologies LTD, Paisley, Scotland, UK) in a humidified atmosphere containing 5% CO₂. SK-BR-3 cells were maintained in McCoy's 5A medium, 518.A2, and Tg1-1 cells in DMEM medium. HTB 132 cells were maintained in Leibovitz's L-15 medium and CO₂ free conditions. All media were purchased from GIBCO, Life Technologies LTD, Paisley, Scotland, UK.

### Preparation of cell lysates

The human breast cancer cell line SK-BR-3 was used as a source of Her-2/neu protein. Tris buffered saline (TBS, pH 7,4) containing 1% Triton X-100 and 1×Complete EDTA free protease inhibitor mix (Roche, Mannheim, Germany) was used for cell lysis. Approximately 30 x 10⁶ SK-BR-3 cells were suspended in one ml of lysis buffer, extensively vortexed, and incubated on ice for 15 min. After disruption, samples were centrifuged 10 min at 800g at room temperature. Supernatants were removed from cell debris and stored at -80°C until use. Before use cell lysates were diluted 1:3 with 0.1 M TBS pH 7.4 and filtered through 0.45 µm.

### Immunoprecipitation

Aliquots of the cell lysat containing 3.5 mg protein were incubated with 40 µl pooled mice sera or 1 µg anti-c-erbB-2 monoclonal antibody (Zymed, San Francisco, CA) and 20 µl protein A+G agarose (Oncogene, Uniondale, NY) and incubated overnight at 4°C. Immunprecipitates were pelleted and washed twice in O,1M TBS and in the same buffer containing 0.5% Nonidet P40. Immunoprecipitated proteins were then separated by 6% SDS-PAGE and analysed by Western blotting.

### Western blot analysis

Precipitates were separated on 6% SDS-PAGE gels and blotted onto nitrocellulose membranes (Schleicher & Schuell, Dassel, Germany). Western blots were performed using 40 mM Na2HPO4, 7 mM NaH2PO4, 1% milk powder, 0.05% w/v sodium azide, 0.5% w/v Tween-20, pH 7.5 for blocking, washing and antibody dilutions. For detection of precipitated Her-2/neu membranes were incubated with rabbit anti-human Her-2/neu antibodies (diluted 1:100; Zymed, San Francisco, CA, USA). Bound rabbit Ig was detected by alkaline phosphatase labeled swine anti-rabbit antibody (diluted 1:500; DAKO A/S, Denmark). The substrate 5-bromo-4-chloro-3-indolyl phosphate/nitro blue tetrazolium was converted in situ into a dense blue compound by immunolocalized alkaline phosphatase.

It could be demonstrated that only the three inventive peptides have the capacity to induce specific anti-her-2/neu immune responses (see Fig. 3)

### Microsomal preparations

Cells were disrupted in lysis buffer (50 mM Na-phosphate ph 7.4, 2 mM EDTA, 250 mM saccharose, 1 X Complete protease inhibitor mix (Roche, Mannheim, Germany) using a dounce tissue grindler. Unbroken nuclei were separated by centrifugation at 1,000 x g. Thereafter supernatants were centrifuged at 32,000g for 1 hr at 4°C. Pellets were solubilized in buffer cotaining 100 mM Na-phosphate pH 7.4, 500 mM NaCl, 2 mM EDTA and 1% Triton X-100. Protein contents were estimated using DC Assay (Bio-Rad Laboraties, Hercules, CA) according to manufacturer's instructions. Samples were stored at 4 °C until use.

### ELISA

### a) Peptide specific Ab responses

96 well microtiter plates (Nunc-Immuno Plate, Nalge Nunc International, Denmark) were coated with 5 µg/ml peptides conjugated to KLH or 5 µg/ml KLH in 100 mM carbonate buffer, pH 9.6 overnight at 4°C. Nonspecific binding sites were blocked for 4 hr with PBS containing 3% milk powder. Sera from mice immunized with peptide-TT conjugates were diluted 1:100 in PBS-tw (0,05% v/v Tween 20) containing 0,5% milk powder, added to antigen-coated plates and incubated overnight at 4°C. Sera from mice immunized with peptide-TT conjugates and IL-12 as additional adjuvant were diluted 1:200-1:4000 for measurement of specific IgG2a and 1:8000-1:100.000 for specific IgG1.

Antibody titers in sera of mice immunized i.n. with peptides-CTB conjuagtes were measured as described above. IgG1 were detected in sera diluted 1:10.000 and IgG2a at a dilation 1:2000. Rat anti-mouse IgG2a or IgG1 (Pharmingen, San Diego, CA,USA) diluted 1:500 in PBS-tw containing 0,5% milk powder were added to the plates. Bound rat Ig was detected with mouse anti-rat HRP conjugated antibodies (Jackson Immunolab, West Grove, Pa, USA), diluted 1:2000 in PBS-tw containing 0,5% milk powder. Color development was performed with TMB substrate (R&D Systems, MN, USA), the reaction was stopped with 0.1 M H2SO4, and the optical density was measured at 450 nm (630 nm as reference wavelength).

### b) Her-2/neu specific Ab responses

Coating with 5 µg/ml mAB Trastuzumab and blocking of nonspecific binding sites were performed as described above. 96 well plates were then incubated with 50 µg/well microsomal preparation of SK-BR-3 or HTB 132 cells diluted in PBS containing 0.5% Triton X-100 for 2 hr at RT. After washing and blocking, plates were incubated overnight at 4°C with mouse antisera diluted 1:250 in PBS. Bound Ig was detected with HRP labeled sheep anti-mouse Ig diluted 1:500 in PBS (Amersham Life Science, Buckinghamshire, England). Colour development was performed as described. OD value for each sample are presented after subtraction of control values.

The inventive vaccine induces a significant antibody response to human HER-2/neu in contrast to the control serum (sera of mice immunized with tetanus-toxoid) (Fig. 4) . After immunization with the inventive vaccine and IL-12 a tendency towards increased antibody levels of these isotypes was observed.

Also the mucosal application of the peptide/CTB conjugates led to induction of specific antibody responses. Both IgG1 and IgG2a antibodies were induced. It could be demonstrated that the mucosal delivery system has the capacity to induce Th1-like immune responses. The data show that mucosal adjuvants, such as CTB, can induce systemic Th1- biased immune responses and also local IgA. The effects are only achieved, if the peptides are conjugated to CTB and not only admixed to the mucosal adjuvant (Fig. 5 a to c).

### Cell proliferation assay

Tumor cells were seeded in 96-well microtiter plates (Costar, Coming, NY) at an optimal density for linear growth: 1×10⁴ cells/well for SK-BR-3 cells and 5x10³/well for 518A2 cells. Cells were allowed to adhere overnight at 37°C. Total IgG isolated from sera of mice and rabbits immunizated with single (fig. 6) or mixed (fig. 7, 8) Her-2/neu peptide conjugates or TT was added at a concentration of 150 µg/ml (Fig. 6) or an increasing concentrations (0,18.75, 37.5, 75 µg/ml) (Fig.7,8) and Trastuzumab, a humanized anti-Her-2/neu IgG1 mAb purchased from Roche (Hertfordshire, UK), at a concentration of 50 µg/ml. Cells were incubated for 72 h at 37°C, thereafter pulsed for 16 h at 37°C with 0.5 µCi [3H]-thymidine/well (Perkin Elmer Life Sciences, Boston, MA) and afterwards harvested. Incorporated [3H]-thymidin was measured in a 1205 Betaplate Liquid Scintillation Counter (Wallac Oy, Turku, Finland). Percentage of inhibition of proliferation was calculated by comparing the cpm values of treated cells with those of untreated cells, which were put at 100%.

Figure 6 shows that antibodies induced after immunization with 2 peptides (ID SEQ 2 and 3) or only with 1 peptide (ID SEQ 1) can induce a 20 % reduction of the growth of tumor cells in vitro; in comparison, Trastuzumab, leads to 40 % tumor growth inhibition in vitro.

Figure 7 shows that antibodies derived from immunization with a mixture with all three inventive peptides induce a more than 60 % tumor growth inhibition, which is even stronger than that induced by the monoclonal ab Trastuzumab. These data show that the combination of the three peptides is of great advantage for the effectiveness of the peptide vaccine and is superior to immunzation with single peptides or combinations of only two peptides.

Furthermore, it could be demonstrated that the proliferation of the SK-BR-3 cells expressing high levels of HER-2/neu was inhibited in a dose dependent manner by the peptide specific antibodies ranging from 39% to 66 % at 18,75 µg/ml to 75 µg/ml antibody response, respectively (Fig. 8).

### Flow cytometry

Tgl-1 cells were stripped from culture flasks and washed in staining buffer consisting of HBSS (GIBCO, Life Technologies LTD) and 5% heat-inactivated FCS. 5×10⁶ cells were incubated with 50 µl of pooled immune sera diluted 1:5 in the staining buffer or with 10 µg of the anti-c-erbB-2 monoclonal antibody (NeoMarkers, Fremont, CA) in the same buffer. After incubation, cells were washed with the staining buffer and stained with FITC-conjugated bovine anti-mouse antibodies (Santa Cruz, CA, USA) diluted 1:100 in the same buffer. All incubations were performed on ice for 45 minutes. After the final washing cells were resuspended in HBSS/0.5% FCS. The fluorescence was measured by a FACScan (Becton Dickinson). The mean channel fluorescence intensity and the percentage of positive cells were determined using the CellQuestTM Pro software.

FACS analysis revealed that sera of mice immunized with the inventive vaccine with or without IL-12 were able to bind the c-neu expressing Tgl-1 cells similarly as a monoclonal control Ab directed both against the human and rat HER-2/neu. Weak staining of the cells was also observed using a pool of sera from TT immunized mice while no binding to Tg1-1 with FITC-conjugated anti-mouse IgG antibody was detected (data not shown).

C-neu specificity of the induced antibodies was further proven by the ability to immunoprecipitate c-neu protein from tumor lysates. In contrast , control sera obtained from naive mice and mice immunized with TT did not show any reactivity with c-neu (data not shown).

### Measurement of cytokine release in vitro

A separate experiment in BALB/mice was performed to study the in vitro cytokine production: Spleens were removed under sterile conditions and prepared as known in the art. Briefly, spleens were homogenized and splenocytes were stimulated with the inventive peptide-vaccine at a concentration of 25 µg/well or with TT at a concentration of 5 µg/well. Control wells were cultured with medium only. Supernatants were collected after 46 h and kept frozen until analysis. IFN-γ levels were measured by ELISA as(not shown). IL-2 and IL-4 levels were measured with commercial mouse ELISA kits (Endogen, Woburn, MA, USA).

As significant amounts of IFN-g were measured in spleen cell cultures of mice immunized with peptides+ IL-12, it could be demonstrated that IL-12 induces INF-gamma production and plays a central role in the improvement of the multiepitope vaccine (Fig. 9).

### Statistical analysis

Comparisons of tumor free interval for all groups were done by a generalization of Gehan's Wilcoxon test. Group differences for time to tumor volume of 1000 mm³ were analysed by Kruskal-Wallis tests. Post hoc comparisons for all pairs of groups were performed applying Tukey-Kramer tests. The same procedure was applied for cytokines. A p-value below 0.05 was considered significant.

In summary, it could be demonstrated that the inventive vaccine, i.e. the multiepitop vaccine, is effective in preventing c-neu overexpressing tumors in vivo and that this effect could be increased by co-administration of IL-12. The induction of a strong immunity by the inventive vaccine leads to an establishment of immunological memory, potentially preventing tumor recurrence. By extrapolation of the present results active immunization with such a multiepitope vaccine, a prophylactic efficacy but also a good therapeutic efficacy against minimal disease of rapidly growing, drug-resistant tumors is expected. Furthermore, the present vaccine could also be administrated as mucosal vaccine without loosing its high immunization activity, which is an attractive vaccine form for all tumors located at mucosal surfaces.

## Claims

1. A vaccine against cancerous diseases associated with the HER-2/neu oncogene, wherein said vaccine comprises a mixture of at least three different peptides having a length of 9 to 30 amino acids and each sequence occurs in the extracellular domain of HER-2/neu protein, wherein at least one peptide has the sequence 378 to 394 of the extracellular domain of HER-2/neu protein, at least another one has the sequence 545 to 560 of the extracellular domain of HER-2/neu protein and at least a further peptide has the sequence 610 to 623 of the extracellular domain of HER-2/neu protein and wherein each of the peptides is conjugated to a carrier.

2. Vaccine against cancerous diseases associated with the HER-2/neu oncogene according to claim 1, wherein at least one peptide having the sequence 378 to 394 of the extracellular domain of HER-2/neu protein is coupled to an additional glycine linker and/or a C-terminal cysteine residue.

3. Vaccine against cancerous diseases associated with the HER-2/neu oncogene according to any of the preceding claims wherein each peptide is conjugated to a carrier in a single or multiple way.

4. Vaccine against cancerous diseases associated with the HER-2/neu oncogene according to claim 3 wherein the carrier is immunogenic.

5. Vaccine against cancerous diseases associated with the HER-2/neu oncogene according to claims 3 or 4 wherein the carrier is selected from the group consisting of keyhole limpet hemocyanin (KLH), tetanus toxoid (TT), B subunit of cholera toxin (CT, CTB), heatlabile toxin (LT) or mutants or B subunit (LTB) of E. coli, bacterial ghosts, liposome, chitosomes , virosomes or dendritic cell.

6. Vaccine against cancerous diseases associated with the HER-2/neu oncogene according to any of the preceding claims wherein the vaccine comprises an adjuvant.

7. Vaccine against cancerous diseases associated with the HER-2/neu oncogene according to any preceding claims wherein the vaccine comprises a mucosal adjuvant.

8. Vaccine against cancerous diseases associated with the HER-2/neu oncogene according to claim 7 wherein the mucosal adjuvant is cholera toxin subunit B (CTB), a lactic acid bacteria or mutant thereof.

9. Vaccine against cancerous diseases associated with the HER-2/neu oncogene according to claims 7 or 8 wherein mucosal adjuvant is used as carrier.

10. Vaccine against cancerous diseases associated with the HER-2/neu oncogene according to any of the preceding claims wherein the mucosal carrier is a probiotic lactic acid bacteria with IL-12 promoting/Th1 activating capacity for oral/mucosal application.

11. Vaccine against cancerous diseases associated with the HER-2/neu oncogene according to any of the preceding claims wherein the vaccine additional comprises interleukine 12 or an IL-12 agonist or a substance that promotes IL-12 production.

12. Use of the vaccine according to claims 1 to 11 for the preparation of a medicament for administrating to a mammal so as to sustain its biological response in the treatment of cancerous diseases associated with the HER-2/neu oncogene, wherein the administration pattern of the medication comprises the steps:
a. administration of said vaccine 4 times in 14 to 21 day intervals and
b. subsequent administration/coadministration of interleukine 12 or an IL-12 agonist or a substance that promotes IL-12 production in a five-day-course, wherein IL-12 is given in two different concentrations.

## Patentansprüche

1. Impfstoff gegen Krebserkrankungen, die mit dem Onkogen HER-2/neu verbunden sind, wobei dieser Impfstoff ein Gemisch von mindestens drei verschiedenen Peptiden mit einer Länge von 9 bis 30 Aminosäuren umfaßt und jede Sequenz in der extrazellulären Domäne des HER-2/neu-Proteins vorkommt, wobei zumindest ein Peptid die Sequenz 378 bis 394 der extrazellulären Domäne des HER-2/neu-Proteins aufweist, zumindest ein anderes die Sequenz 545 bis 560 der extrazellulären Domäne des HER-2/neu-Proteins und zumindest ein weiteres Peptid die Sequenz 610 bis 623 der extrazellulären Domäne des HER-2/neu-Proteins hat, und wobei jedes der Peptide mit einem Träger konjugiert ist.

2. Impfstoff gegen Krebserkrankungen, die mit dem Onkogen HER-2/neu verbunden sind, nach Anspruch 1, wobei zumindest ein Peptid mit der Sequenz 378 bis 394 der extrazellulären Domäne des HER-2/neu-Proteins an einen zusätzlichen Glycin-Linker und/oder einen C-terminalen Cysteinrest gekoppelt ist.

3. Impfstoff gegen Krebserkrankungen, die mit dem Onkogen HER-2/neu verbunden sind, nach einem der vorstehenden Ansprüche, wobei jedes Peptid in einfacher oder mehrfacher Art und Weise mit einem Träger konjugiert ist.

4. Impfstoff gegen Krebserkrankungen, die mit dem Onkogen HER-2/neu verbunden sind, nach Anspruch 3, wobei der Träger immunogen ist.

5. Impfstoff gegen Krebserkrankungen, die mit dem Onkogen HER-2/neu verbunden sind, nach den Ansprüchen 3 oder 4, wobei der Träger aus der Gruppe ausgewählt ist, bestehend aus Keyhole-Limpet-Hämocyanin (KLH), Tetanus-Toxoid (TT), der B-Untereinheit von Cholera-Toxin (CT, CTB), wärmeempfindlichem Toxin (LT) oder Mutanten oder einer B-Untereinheit (LTB) von E. coli, Bakterien-Ghosts, Liposom, Chitosomen, Virosomen oder einer dendritischen Zelle.

6. Impfstoff gegen Krebserkrankungen, die mit dem Onkogen HER-2/neu verbunden sind, nach einem der vorstehenden Ansprüche, wobei der Impfstoff ein Adjuvans umfaßt.

7. Impfstoff gegen Krebserkrankungen, die mit dem Onkogen HER-2/neu verbunden sind, nach einem der vorstehenden Ansprüche, wobei der Impfstoff ein mukosales Adjuvans umfaßt.

8. Impfstoff gegen Krebserkrankungen, die mit dem Onkogen HER-2/neu verbunden sind, nach Anspruch 7, wobei das mukosale Adjuvans die B-Untereinheit von Cholera-Toxin (CTB), ein Milchsäurebakterium oder eine Mutante davon ist.

9. Impfstoff gegen Krebserkrankungen, die mit dem Onkogen HER-2/neu verbunden sind, nach den Ansprüchen 7 oder 8, wobei das mukosale Adjuvans als Träger verwendet wird.

10. Impfstoff gegen Krebserkrankungen, die mit dem Onkogen HER-2/neu verbunden sind, nach einem der vorstehenden Ansprüche, wobei der mukosale Träger ein probiotisches Milchsäurebakterium mit einer IL-12 fördernden/Th1 aktivierenden Leistungsfähigkeit für die orale/mukosale Anwendung ist.

11. Impfstoff gegen Krebserkrankungen, die mit dem Onkogen HER-2/neu verbunden sind, nach einem der vorstehenden Ansprüche, wobei der Impfstoff außerdem Interleukin 12 oder einen IL-12-Agonisten oder eine Substanz umfaßt, die die Produktion von IL-12 fördert.

12. Verwendung des Impfstoffs nach den Ansprüchen 1 bis 11 für die Herstellung eines Medikamentes zur Verabreichung an einen Säuger, so daß dessen biologische Reaktion bei der Behandlung von Krebserkrankungen, die mit dem Onkogen HER 2/neu verbunden sind, aufrechterhalten wird, wobei das Verabreichungsprofil der Medikation die folgenden Schritte umfaßt:
a. Verabreichung des Impfstoffs 4 mal in Intervallen von 14 bis 21 Tagen und
b. anschließende Verabreichung/gleichzeitige Verabreichung von Interleukin 12 oder einem IL-12-Agonisten oder einer Substanz, die die Produktion von IL-12 fördert, in einem fünftägigen Verlauf, wobei IL-12 in zwei unterschiedlichen Konzentrationen gegeben wird.

## Revendications

1. Vaccin contre des maladies cancéreuses associées à l'oncogène HER-2/neu, ledit vaccin comprenant un mélange d'au moins trois peptides différents ayant une longueur de 9 à 30 acides aminés et chaque séquence étant présente dans le domaine extracellulaire de la protéine HER-2/neu, au moins un peptide ayant la séquence 378 à 394 du domaine extracellulaire de la protéine HER-2/neu, au moins un autre ayant la séquence 545 à 560 du domaine extracellulaire de la protéine HER-2/neu, au moins un autre peptide ayant la séquence 610 à 623 du domaine extracellulaire de là protéine HER-2/neu et chacun des peptides étant conjugué à un vecteur.

2. Vaccin contre des maladies cancéreuses associées à l'oncogène HER-2/neu selon la revendication 1, dans lequel au moins un peptide ayant la séquence 378 à 394 du domaine extracellulaire de la protéine HER-2/neu est couplé à un lieur glycine supplémentaire et/ou à un résidu cystéine C-términal.

3. Vaccin contre des maladies cancéreuses associées à l'oncogène HER-2/neu selon l'une quelconque des revendications précédentes, dans lequel chaque peptide est conjugué à un vecteur de manière unique ou multiple.

4. Vaccin contre des maladies cancéreuses associées à l'oncogène HER-2/neu selon la revendication 3 dans lequel le vecteur est immunogène.

5. Vaccin contre des maladies cancéreuses associées à l'oncogène HER-2/neu selon les revendications 3 ou 4 dans lequel le vecteur est choisi parmi le groupe consistant en l'hémocyanine de patelle (KLH), l'anatoxine tétanique (TT), la sous-unité B de la toxine cholérique (CT, CTB), la toxine thermolabile (LT) ou ses mutants ou sa sous-unité B (LTB) de *E. coli*, des bactéries vides, un liposome, des chitosomes, des virosomes ou une cellule dendritique.

6. Vaccin contre des maladies cancéreuses associées à l'oncogène HER-2/neu selon l'une quelconque des revendications précédentes, dans lequel le vaccin comprend un adjuvant.

7. Vaccin contre des maladies cancéreuses associées à l'oncogène HER-2/neu selon l'une quelconque des revendications précédentes, dans lequel le vaccin contient un adjuvant muqueux.

8. Vaccin contre des maladies cancéreuses associées à l'oncogène HER-2/neu selon la revendication 7, dans lequel l'adjuvant muqueux est la sous-unité B de la toxine cholérique (CTB), une bactérie lactique ou un mutant de celle-ci.

9. Vaccin contre des maladies cancéreuses associées à l'oncogène HER-2/neu selon les revendications 7 ou 8 dans lequel l'adjuvant muqueux est utilisé comme vecteur.

10. Vaccin contre des maladies cancéreuses associées à l'oncogène HER-2/neu selon l'une quelconque des revendications précédentes dans lequel le vecteur muqueux est une bactérie lactique probiotique ayant une capacité de promotion de l'IL-12/activation Th1 pour application orale/muqueuse.

11. Vaccin contré des maladies cancéreuses associées à l'oncogène HER-2/neu selon l'une quelconque des revendications précédentes dans lequel le vaccin comprend en outre de l'interleukine 12 ou un agoniste de l'IL-12 ou une substance qui favorise la production d'IL-12.

12. Utilisation du vaccin selon les revendications 1 à 11 pour la préparation d'un médicament destiné à être administré à un mammifère afin de soutenir sa réponse biologique dans le traitement de maladies cancéreuses associées à l'oncogène HER-2/neu, le schéma d'administration du médicament comprenant les étapes consistant à :
a. administrer ledit vaccin 4 fois à intervalles de 14 à 21 jours et
b. ensuite administrer/co-administrer de l'interleukine 12 ou un agoniste de l'IL-12 ou une substance qui favorise la production d'IL-12 pendant cinq jours de traitement, l'IL-12 étant administrée à deux concentrations différentes.


### SEQUENCE LISTING

<110> Bio Life Science Forschungs- und Entwicklungsges.m.b.H.
<120> HER-2/neu multi-peptide vaccine
<130> K 52 448
<160> 3
<170> PatentIn version 3.3
<210> 1
   <211> 17
   <212> PRT
   <213> Artificial
<220>
   <223> HER-2/neu peptide
<400> 1
   Pro Glu Ser Phe Asp Gly Asp Pro Ala Ser Asn Thr Ala Pro Leu Gln
   1               5                   10                  15
   Pro
<210> 2
   <211> 16
   <212> PRT
   <213> Artificial
<220>
   <223> HER-2/neu peptide
<400> 2
   Arg Val Leu Gln Gly Leu Pro Arg Glu Tyr Val Asn Ala Arg His Cys
   1               5                   10                  15
<210> 3
   <211> 14
   <212> PRT
   <213> Artificial
<220>
   <223> HER-2/neu peptide
<400> 3
   Tyr Met Pro Ile Trp Lys Phe Pro Asp Glu Glu Gly Ala Cys
   1               5                   10

### SEQUENCE LISTING

<110> Bio Life Science Forschungs- und Entwicklungsges.m.b.H.
<120> HER-2/neu multi-peptide vaccine
<130> K 52 448
<160> 3
<170> PatentIn version 3.3
<210> 1
   <211> 17
   <212> PRT
   <213> Artificial
<220>
   <223> HER-2/neu peptide
<400> 1
   Pro Glu Ser Phe Asp Gly Asp Pro Ala Ser Asn Thr Ala Pro Leu Gln
   1               5                   10                  15
   Pro
<210> 2
   <211> 16
   <212> PRT
   <213> Artificial
<220>
   <223> HER-2/neu peptide
<400> 2
   Arg Val Leu Gln Gly Leu Pro Arg Glu Tyr Val Asn Ala Arg His Cys
   1               5                   10                  15
<210> 3
   <211> 14
   <212> PRT
   <213> Artificial
<220>
   <223> HER-2/neu peptide
<400> 3
   Tyr Met Pro Ile Trp Lys Phe Pro Asp Glu Glu Gly Ala Cys
   1               5                   10